# EUROPEAN PATENT SPECIFICATION

(11) **EP 0 325 400 B1**
(45) Date of publication and mention of the grant of the patent: **13.04.1994**
(21) Application number: 89300388.9
(22) Date of filing: 17.01.1989
(51) Int. Cl.: C12N 15/32

(54) **Novel hybrid bacillus thuringiensis gene, plasmid, and transformed pseudomonas fluorescens**
Bacillus-thuringiensis-Hybridgen, Plasmid und transformiertes Pseudomonas fluorescens
Gène d'hybride de bacillus thuringiensis, plasmide et pseudomonas fluorescens transformé

(30) Priority: 22.01.1988 US 146997
(43) Date of publication of application: 26.07.1989
(73) Proprietor: MYCOGEN CORPORATION, San Diego, California 92121 (US)
(72) Inventor: Gilroy, Thomas E., San Diego, CA 92126 (US); Wilcox, Edward R., Escondido, CA 92025 (US)
(74) Representative: Perry, Robert Edward

(56) References cited:
- EP-A- 0 193 259
- EP-A- 0 213 818
- EP-A- 0 269 601
- US-A- 4 448 885
- Höfte et al. Biol. Reviews June 1989, p.242-255.

## Description

The most widely used microbial pesticides are derived from the bacterium Bacillus thuringiensis. This bacterial agent is used to control a wide range of leaf-eating caterpillars, Japanese beetles and mosquitos. Bacillus thuringiensis produces a proteinaceous paraspore or crystal which is toxic upon ingestion by a susceptible insect host. For example, B. thuringiensis var. kurstaki HD-1 produces a crystal called a delta toxin which is toxic to the larvae of a number of lepidopteran insects. The cloning and expression of this B.t. crystal protein gene in Escherichia coli has been described in the published literature (Schnepf, H.E. and Whitely, H.R. [1981] Proc. Natl. Acad. Sci. USA 78:2893-2897). U.S. Patent 4,448,885 and U.S. Patent 4,467,036 both disclose the expression of B.t. crystal protein in E. coli. In U.S. 4,467,036 B. thuringiensis var. kurstaki HD-1 is disclosed as being available from the well-known NRRL culture repository at Peoria, Illinois. Its accession number there is NRRL B-3792. B. thuringiensis var. kurstaki HD-73 is also available from NRRL. Its accession number is NRRL B-4488.

### Brief Summary of the Invention

Disclosed and claimed is a novel hybrid toxin gene toxic to lepidopteran insects. This toxin gene has been transferred to a Pseudomonas fluorescens host via a plasmid vector.

Specifically, the invention comprises a novel hybrid delta endotoxin gene comprising part of the B. thuringiensis var. kurstaki HD-73 toxin gene and part of the toxin gene from B. thuringiensis var. kurstaki strain HD-1. This hybrid gene was inserted into a suitable transfer vector which was then used to transform a Pseudomonas fluorescens host. The P. fluorescens host can be used as an insecticide active against lepidopteran insects.

More specifically, the subject invention concerns a novel hybrid toxin gene (DNA) encoding a novel protein having activity against lepidopteran insects.

### Detailed Disclosure of the Invention

The novel hybrid toxin gene of the subject invention comprises part of the B. thuringiensis var. kurstaki HD-73 toxin gene and part of a B. thuringiensis var. kurstaki strain HD-1 toxin gene. In general, the B.t.k. HD-73 gene portion was initially combined with DNA segments derived from (1) the known E. coli plasmid pBR322, (2) a DNA segment conferring the ability to replicate in Pseudomonas from the known plasmid pRO1614, and (3) a DNA segment representing the hybrid Tac promoter.

The resulting hybrid gene was contained in a plasmid named pM2,16-11. This plasmid was used to transform a Pseudomonas fluorescens microbe to give the transformed strain named MR436.

The toxin gene of the subject invention can be introduced into a wide variety of microbial hosts. Expression of the toxin gene results, directly or indirectly, in the intracellular production and maintenance of the pesticide. With suitable hosts, e.g., Pseudomonas, the microbes can be applied to the situs of lepidopteran insects where they will proliferate and be ingested by the insects. The result is a control of the unwanted insects. Alternatively, the microbe hosting the toxin gene can be treated under conditions that prolong the activity of the toxin produced in the cell. The treated cell then can be applied to the environment of target pest(s). The resulting product retains the toxicity of the B.t. toxin.

Where the B.t. toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Pseudomonas, Erwinia, Serratia, Klebsiella, Xanthomonas, Streptomyces, Rhizobium, Rhodopseudomonas, Methylophilius, Agrobacterium, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, and Aureobasidium. Of particular interest are such phytosphere bacterial species as Pseudomonas syringae. Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae, and Aureobasidium pollulans. Of particular interest are the pigmented microorganisms.

A wide variety of ways are available for introducing the B.t. gene expressing the toxin into the microorganism host under conditions which allow for stable maintenance and expression of the gene. One can provide for DNA constructs which include the transcriptional and translational regulatory signals for expression of the toxin gene, the toxin gene under their regulatory control and a DNA sequence homologous with a sequence in the host organism, whereby integration will occur, and/or a replication system which is functional in the host, whereby integration or stable maintenance will occur.

The transcriptional initiation signals will include a promoter and a transcriptional initiation start site. In some instances, it may be desirable to provide for regulative expression of the toxin, where expression of the toxin will only occur after release into the environment. This can be achieved with operators or a region binding to an activator or enhancers, which are capable of induction upon a change in the physical or chemical environment of the microorganisms. For example, a temperature sensitive regulatory region may be employed, where the organisms may be grown up in the laboratory without expression of a toxin, but upon release into the environment, expression would begin. Other techniques may employ a specific nutrient medium in the laboratory, which inhibits the expression of the toxin, where the nutrient medium in the environment would allow for expression of the toxin. For translational initiation, a ribosomal binding site and an initiation codon will be present.

Various manipulations may be employed for enhancing the expression of the messenger, particularly by using an active promoter, as well as by employing sequences, which enhance the stability of the messenger RNA. The initiation and translational termination region will involve stop codon(s), a terminator region, and optionally, a polyadenylation signal.

In the direction of transcription, namely in the 5′ to 3′ direction of the coding or sense sequence, the construct will involve the transcriptional regulatory region, if any, and the promoter, where the regulatory region may be either 5′ or 3′ of the promoter, the ribosomal binding site, the initiation codon, the structural gene having an open reading frame in phase with the initiation codon, the stop codon(s), the polyadenylation signal sequence, if any, and the terminator region. This sequence as a double strand may be used by itself for transformation of a microorganism host, but will usually be included with a DNA sequence involving a marker, where the second DNA sequence may be joined to the toxin expression construct during introduction of the DNA into the host.

By a marker is intended a structural gene which provides for selection of those hosts which have been modified or transformed. The marker will normally provide for selective advantage, for example, providing for biocide resistance, e.g., resistance to antibiotics or heavy metals; complementation, so as to provide prototrophy to an auxotrophic host, or the like. Preferably, complementation is employed, so that the modified host may not only be selected, but may also be competitive in the field. One or more markers may be employed in the development of the constructs, as well as for modifying the host. The organisms may be further modified by providing for a competitive advantage against other wild-type microorganisms in the field. For example, genes expressing metal chelating agents, e.g., siderophores, may be introduced into the host along with the structural gene expressing the toxin. In this manner, the enhanced expression of a siderophore may provide for a competitive advantage for the toxin-producing host, so that it may effectively compete with the wild-type microorganisms and stably occupy a niche in the environment.

Where no functional replication system is present, the construct will also include a sequence of at least 50 basepairs (bp), preferably at least about 100 bp, and usually not more than about 1000 bp of a sequence homologous with a sequence in the host. In this way, the probability of legitimate recombination is enhanced, so that the gene will be integrated into the host and stably maintained by the host. Desirably, the toxin gene will be in close proximity to the gene providing for complementation as well as the gene providing for the competitive advantage. Therefore, in the event that a toxin gene is lost, the resulting organism will be likely to also lose the complementing gene and/or the gene providing for the competitive advantage, so that it will be unable to compete in the environment with the gene retaining the intact construct.

A large number of transcriptional regulatory regions are available from a wide variety of microorganism hosts, such as bacteria, bacteriophage, cyanobacteria, algae, fungi, and the like. Various transcriptional regulatory regions include the regions associated with the trp gene, lac gene, gal gene, the lambda left and right promoters, the Tac promoter, the naturally-occurring promoters associated with the toxin gene, where functional in the host. See for example, U.S. Patent Nos. 4,332,898, 4,342,832 and 4,356,270. The termination region may be the termination region normally associated with the transcriptional initiation region or a different transcriptional initiation region, so long as the two regions are compatible and functional in the host.

Where stable episomal maintenance or integration is desired, a plasmid will be employed which has a replication system which is functional in the host. The replication system may be derived from the chromosome, an episomal element normally present in the host or a different host, or a replication system from a virus which is stable in the host. A large number of plasmids are available, such as pBR322, pACYC184, RSF1010, pRO1614, and the like. See for example, Olson et al., (1982) J. Bacteriol. 150:6069, and Bagdasarian et al., (1981) Gene 16:237, and U.S. Patent Nos. 4,356,270, 4,362,817, and 4,371,625.

The B.t. gene can be introduced between the transcriptional and translational initiation region and the transcriptional and translational termination region, so as to be under the regulatory control of the initiation region. This construct will be included in a plasmid, which will include at least one replication system, but may include more than one, where one replication system is employed for cloning during the development of the plasmid and the second replication system is necessary for functioning in the ultimate host. In addition, one or more markers may be present, which have been described previously. Where integration is desired, the plasmid will desirably include a sequence homologous with the host genome.

The transformants can be isolated in accordance with conventional ways, usually employing a selection technique, which allows for selection of the desired organism as against unmodified organisms or transferring organisms, when present. The transformants then can be tested for pesticidal activity.

Suitable host cells, where the pesticide-containing cells will be treated to prolong the activity of the toxin in the cell when the then treated cell is applied to the environment of target pest(s), may include either prokaryotes or eukaryotes, normally being limited to those cells which do not produce substances toxic to higher organisms, such as mammals. However, organisms which produce substances toxic to higher organisms could be used, where the toxin is unstable or the level of application sufficiently low as to avoid any possibility of toxicity to a mammalian host. As hosts, of particular interest will be the prokaryotes and the lower eukaryotes, such as fungi. Illustrative prokaryotes, both Gram-negative and -positive, include Enterobacteriaceae, such as Escherichia, Erwinia, Shigella, Salmonella, and Proteus; Bacillaceae; Rhizobiceae, such as Rhizobium; Spirillaceae, such as photobacterium, Zymomonas, Serratia, Aeromonas, Vibrio, Desulfovibrio, Spirillum; Lactobacillaceae; Pseudomonadaceae, such as Pseudomonas and Acetobacter; Azotobacteraceae and Nitrobacteraceae. Among eukaryotes are fungi, such as Phycomycetes and Ascomycetes, which includes yeast, such as Saccharomyces and Schizosaccharomyces; and Basidiomycetes yeast, such as Rhodotorula, Aureobasidium, Sporobolomyces, and the like.

Characteristics of particular interest in selecting a host cell for purposes of production include ease of introducing the B.t.i. gene into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; leaf affinity; lack of mammalian toxicity; attractiveness to pests for ingestion; ease of killing and fixing without damage to the toxin; and the like. Other considerations include ease of formulation and handling, economics, storage stability, and the like.

Host organisms of particular interest include yeast, such as Rhodotorula sp., Aureobasidium sp., Saccharomyces sp., and Sporobolomyces sp.; phylloplane organisms such as Pseudomonas sp., Erwinia sp. and Flavobacterium sp.; or such other organisms as Escherichia, Lactobacillus sp., Bacillus sp., and the like. Specific organisms include Pseudomonas aeruginosa, Pseudomonas fluorescens, Saccharomyces cerevisiae, Bacillus thuringiensis, Escherichia coli, Bacillus subtilis, and the like.

The cell will usually be intact and be substantially in the proliferative form when treated, rather than in a spore form, although in some instances spores may be employed.

Treatment of the microbial cell, e.g., a microbe containing the B.t. toxin gene, can be by chemical or physical means, or by a combination of chemical and/or physical means, so long as the technique does not deleteriously affect the properties of the toxin, nor diminish the cellular capability in protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as formaldehyde and glutaraldehyde; anti-infectives, such as zephiran chloride and cetylpyridinium chloride; alcohols, such as isopropyl and ethanol; various histologic fixatives, such as Bouin's fixative and Helly's fixative (See: Humason, Gretchen L., Animal Tissue Techniques, W.H. Freeman and Company, 1967); or a combination of physical (heat) and chemical agents that preserve and prolong the activity of the toxin produced in the cell when the cell is administered to the host animal. Examples of physical means are short wavelength radiation such as gamma-radiation and X-radiation, freezing, UV irradiation, lyophilization, and the like.

The cells generally will have enhanced structural stability which will enhance resistance to environmental conditions. Where the pesticide is in a proform, the method of inactivation should be selected so as not to inhibit processing of the proform to the mature form of the pesticide by the target pest pathogen. For example, formaldehyde will crosslink proteins and could inhibit processing of the proform of a polypeptide pesticide. The method of inactivation or killing retains at least a substantial portion of the bio-availability or bioactivity of the toxin.

The cellular host containing the B.t. insecticidal gene may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells may be formulated in a variety of ways. They may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

The pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about 10² to about 10⁴ cells/mg. These formulations will be administered at about 50 mg (liquid or dry) to 1 kg or more per hectare.

The formulations can be applied to the environment of the lepidopteran pest(s), e.g., plants, soil or water, by spraying, dusting, sprinkling, or the like.

Following are examples which illustrate procedures, including the best mode, for practicing the invention. These examples should not be construed as limiting. All percentages are by weight and all solvent mixture proportions are by volume unless otherwise noted.

### Example 1 - Construction of Novel Hybrid Toxin Gene and Transformation into Pseudomonas fluorescens

A portion of the B. thuringiensis var. kurstaki HD-73 gene including all of the toxin-encoding DNA from the starting ATG (i.e., start methionine) to the HindIII site was inserted into the Tac-promoted plasmid pKK223-3 (Pharmacia). This was done by making a blunt fusion of this gene just downstream from the ribosome binding site in pKK223-3. This formed plasmid pKK2. Next, the 3′ portion of a toxin gene from the Berliner strain of B.t. (DNA 5:305-314 [1986]) was cloned as a SacI to PstI fragment into pKK2/SacI + PstI thus making a recombinant toxin gene in a new plasmid named pKK73BB-9. This gene is of Berliner origin (DNA 5:305-314, 1986) for all sequences beyond (3′ to) the SacI site.

Next, pKK73BB-9 was cleaved with NsiI, to release the internal portion of the toxin DNA, treated with bacterial alkaline phosphatose, gel purified and used to subclone a partial HD73-like toxin gene fragment with NsiI termini. The resulting plasmid is called pKK1-73. The HD73-like fragment represents all the DNA sequences between the two NsiI sites commonly found in B.t. toxins. Next, pKK1-73 was cleaved with Tth111I, made blunt with treatment with Klenow fragment plus dNTPs, linkered for BamH1, BamH1 digested, ligated, transformed, and screened to find a plasmid which had deleted the partial tetracycline gene (derived from pKK223-3). This resulted in plasmid p1,123-1. Plasmid p1,123-1 was cleaved with PvuI, treated briefly with Bal 31, made blunt with Klenow and used to clone the similarly blunted tetracycline resistance gene from pBR322 (map positions EcoR1 to AvaI). Screening of tetracycline resistant colonies produced the desired plasmid p1,130-6. This plasmid was linearized by partial digestion with BamH1, gel purified and was used to receive the BamH1 terminated Pseudomonas origin of replication from pRO1614. The final result is plasmid pM2,16-11.

Plasmid pM2,16-11 was used to transform a Pseudomonas fluorescens using standard transformation procedures.

The HD73-like NsiI fragment, discussed above, was constructed as follows. To the 3′ end of the HindIII-terminated partial HD73 gene was added a HindIII to NdeI Berliner-like toxin sequence cloned from B. thuringiensis var. kurstaki HD-1.

The above cloning procedures were conducted using standard procedures unless otherwise noted.

The various methods employed in the preparation of the plasmids and transformation of host organisms are well known in the art. These procedures are all described in Maniatis et al (1982) "Molecular Cloning: A Laboratory Manual", Cold Spring Harbor Laboratory, New York. It is within the skill of those in the genetic engineering art to extract DNA from microbial cells, perform restriction enzyme digestions, electrophorese DNA fragments, tail and anneal plasmid and insert DNA, ligate DNA, transform cells, prepare plasmid DNA, electrophorese proteins, and sequence DNA.

The restriction enzymes disclosed herein can be purchased from Bethesda Research Laboratories, Gaithersburg, Maryland, USA, or New England Biolabs, Beverly, Massachusetts, USA. The enzymes are used according to the instructions provided by the supplier.

PLasmid pM2,16-11, containing the B.t. toxin gene, can be removed from the transformed host microbe by use of standard well known procedures. For example, P. fluorescens (pM2,16-11) can be subjected to cleared lysate isopcynic density gradient procedures, to recover pM2,16-11.

A subculture of P. fluorescens (pM2,16-11) was deposited in the permanent collection of the Agricultural Research Service Patent Culture Collection (NRRL), Regional Research Center, 1815 North University Street, Peoria, Illinois 61604, USA on 15.01.88. The accession number is NRRL B-18292.

### Example 2 - Insertion of Toxin Gene into Plants

The novel gene can be inserted into plant cells using the Ti plasmid from Agrobacter tumefaciens. Plant cells can then be caused to regenerate into plants; see Zambryski et al (1983) Cell 32:1033-1043. A particularly useful vector in this regard is pEND4K; see Klee et al (1985) Biotechnology 3:637-642. This plasmid can replicate both in plant cells and in bacteria and has multiple cloning sites for passenger genes. The toxin gene, for example, can be inserted into the BamHI site of pEND4K, propagated in E. coli, and transformed into appropriate plant cells.

### Example 3 - Cloning of Novel Hybrid B. thuringiensis Genes into Baculoviruses

The novel hybrid gene can be cloned into baculoviruses such as Autographa californica nuclear polyhedrosis virus (AcNPV). Plasmids can be constructed that contain the AcNPV genome cloned into a commercial cloning vector such as pUC8. The AcNPV genome is modified so that the coding region of the polyhedrin gene is removed and a unique cloning site for a passenger gene is placed directly behind the polyhedrin promoter. Examples of such vectors are pGP-B6874, described by Pennock et al. Mol. Cell. Biol. 4:399-406, and pAC380, described by Smith et al (1983) Mol. Cell. Biol. 3:2156-2165. The gene coding for the novel protein toxin of the invention can be modified with BamHI linkers at appropriate regions both upstream and downstream from the coding region and inserted into the passenger site of one of the AcNPV vectors.

The particular nucleotide sequence encoding the novel B.t. toxin gene, and the deduced amino-acid sequence are shown in claim 1.

It is well known in the art that the amino-acid sequence of a protein is determined by the nucleotide sequence of the DNA. Because of the redundancy of the genetic code, i.e. more than one coding nucleotide triplet (codon) can be used for most of the amino-acids used to make proteins, different nucleotide sequences can code for a particular amino-acid.

The novel B.t. toxin can be prepared via any nucleotide sequence (equivalent to that shown) encoding the same amino-acid sequence; the present invention includes such equivalent nucleotide sequences.

### Chart:

Chart:

5 10 15 20
Met Asp Asn Asn Pro Asn Ile Asn Glu Cys Ile Pro Tyr Asn Cys Leu Ser Asn Pro Glu
ATG GAT AAC AAT CCG AAC ATC AAT GAA TGC ATT CCT TAT AAT TGT TTA AGT AAC CCT GAA

25 30 35 40
Val Glu Val Leu Gly Gly Glu Arg Ile Glu Thr Gly Tyr Thr Pro Ile Asp Ile Ser Leu
GTA GAA GTA TTA GGT GGA GAA AGA ATA GAA ACT GGT TAC ACC CCA ATC GAT ATT TCC TTG

45 50 55 60
Ser Leu Thr Gln Phe Leu Leu Ser Glu Phe Val Pro Gly Ala Gly Phe Val Leu Gly Leu
TCG CTA ACG CAA TTT CTT TTG AGT GAA TTT GTT CCC GGT GCT GGA TTT GTG TTA GGA CTA

65 70 75 80
Val Asp Ile Ile Trp Gly Ile Phe Gly Pro Ser Gln Trp Asp Ala Phe Leu Val Gln Ile
GTT GAT ATA ATA TGG GGA ATT TTT GGT CCC TCT CAA TGG GAC GCA TTT CTT GTA CAA ATT

85 90 95 100
Glu Gln Leu Ile Asn Gln Arg Ile Glu Glu Phe Ala Arg Asn Gln Ala Ile Ser Arg Leu
GAA CAG TTA ATT AAC CAA AGA ATA GAA GAA TTC GCT AGG AAC CAA GCC ATT TCT AGA TTA

105 110 115 120
Glu Gly Leu Ser Asn Leu Tyr Gln Ile Tyr Ala Glu Ser Phe Arg Glu Trp Glu Ala Asp
GAA GGA CTA AGC AAT CTT TAT CAA ATT TAC GCA GAA TCT TTT AGA GAG TGG GAA GCA GAT

125 130 135 140
Pro Thr Asn Pro Ala Leu Arg Glu Glu Met Arg Ile Gln Phe Asn Asp Met Asn Ser Ala
CCT ACT AAT CCA GCA TTA AGA GAA GAG ATG CGT ATT CAA TTC AAT GAC ATG AAC AGT GCC

145 150 155 160
Leu Thr Thr Ala Ile Pro Leu Phe Ala Val Gln Asn Tyr Gln Val Pro Leu Leu Ser Val
CTT ACA ACC GCT ATT CCT CTT TTT GCA GTT CAA AAT TAT CAA GTT CCT CTT TTA TCA GTA

165 170 175 180
Tyr Val Gln Ala Ala Asn Leu His Leu Ser Val Leu Arg Asp Val Ser Val Phe Gly Gln
TAT GTT CAA GCT GCA AAT TTA CAT TTA TCA GTT TTG AGA GAT GTT TCA GTG TTT GGA CAA

185 190 195 200
Arg Trp Gly Phe Asp Ala Ala Thr Ile Asn Ser Arg Tyr Asn Asp Leu Thr Arg Leu Ile
AGG TGG GGA TTT GAT GCC GCG ACT ATC AAT AGT CGT TAT AAT GAT TTA ACT AGG CTT ATT

205 210 215 220
Gly Asn Tyr Thr Asp Tyr Ala Val Arg Trp Tyr Asn Thr Gly Leu Glu Arg Val Trp Gly
GGC AAC TAT ACA GAT TAT GCT GTA CGC TGG TAC AAT ACG GGA TTA GAA CGT GTA TGG GGA

225 230 235 240
Pro Asp Ser Arg Asp Trp Val Arg Tyr Asn Gln Phe Arg Arg Glu Leu Thr Leu Thr Val
CCG GAT TCT AGA GAT TGG GTA AGG TAT AAT CAA TTT AGA AGA GAA TTA ACA CTA ACT GTA

245 250 255 260
Leu Asp Ile Val Ala Leu Phe Pro Asn Tyr Asp Ser Arg Arg Tyr Pro Ile Arg Thr Val
TTA GAT ATC GTT GCT CTG TTC CCG AAT TAT GAT AGT AGA AGA TAT CCA ATT CGA ACA GTT

265 270 275 280
Ser Gln Leu Thr Arg Glu Ile Tyr Thr Asn Pro Val Leu Glu Asn Phe Asp Gly Ser Phe
TCC CAA TTA ACA AGA GAA ATT TAT ACA AAC CCA GTA TTA GAA AAT TTT GAT GGT AGT TTT

285 290 295 300
Arg Gly Ser Ala Gln Gly Ile Glu Arg Ser Ile Arg Ser Pro His Leu Met Asp Ile Leu
CGA GGC TCG GCT CAG GGC ATA GAA AGA AGT ATT AGG AGT CCA CAT TTG ATG GAT ATA CTT

305 310 315 320
Asn Ser Ile Thr Ile Tyr Thr Asp Ala His Arg Gly Tyr Tyr Tyr Trp Ser Gly His Gln
AAC AGT ATA ACC ATC TAT ACG GAT GCT CAT AGG GGT TAT TAT TAT TGG TCA GGG CAT CAA

325 330 335 340
Ile Met Ala Ser Pro Val Gly Phe Ser Gly Pro Glu Phe Thr Phe Pro Leu Tyr Gly Thr
ATA ATG GCT TCT CCT GTA GGG TTT TCG GGG CCA GAA TTC ACT TTT CCG CTA TAT GGA ACT

345 350 355 360
Met Gly Asn Ala Ala Pro Gln Gln Arg Ile Val Ala Gln Leu Gly Gln Gly Val Tyr Arg
ATG GGA AAT GCA GCT CCA CAA CAA CGT ATT GTT GCT CAA CTA GGT CAG GGC GTG TAT AGA

365 370 375 380
Thr Leu Ser Ser Thr Leu Tyr Arg Arg Pro Phe Asn Ile Gly Ile Asn Asn Gln Gln Leu
ACA TTA TCG TCC ACT TTA TAT AGA AGA CCT TTT AAT ATA GGG ATA AAT AAT CAA CAA CTA

385 390 395 400
Ser Val Leu Asp Gly Thr Glu Phe Ala Tyr Gly Thr Ser Ser Asn Leu Pro Ser Ala Val
TCT GTT CTT GAC GGG ACA GAA TTT GCT TAT GGA ACC TCC TCA AAT TTG CCA TCC GCT GTA

405 410 415 420
Tyr Arg Lys Ser Gly Thr Val Asp Ser Leu Asp Glu Ile Pro Pro Gln Asn Asn Asn Val
TAC AGA AAA AGC GGA ACG GTA GAT TCG CTG GAT GAA ATA CCG CCA CAG AAT AAC AAC GTG

425 430 435 440
Pro Pro Arg Gln Gly Phe Ser His Arg Leu Ser His Val Ser Met Phe Arg Ser Gly Phe
CCA CCT AGG CAA GGA TTT AGT CAT CGA TTA AGC CAT GTT TCA ATG TTT CGT TCA GGC TTT

445 450 455 460
Ser Asn Ser Ser Val Ser Ile Ile Arg Ala Pro Met Phe Ser Trp Ile His Arg Ser Ala
AGT AAT AGT AGT GTA AGT ATA ATA AGA GCT CCT ATG TTC TCT TGG ATA CAT CGT AGT GCT

465 470 475 480
Glu Phe Asn Asn Ile Ile Ala Ser Asp Ser Ile Thr Gln Ile Pro Ala Val Lys Gly Asn
GAA TTT AAT AAT ATA ATT GCA TCG GAT AGT ATT ACT CAA ATC CCT GCA GTG AAG GGA AAC

485 490 495 500
Phe Leu Phe Asn Gly Ser Val Ile Ser Gly Pro Gly Phe Thr Gly Gly Asp Leu Val Arg
TTT CTT TTT AAT GGT TCT GTA ATT TCA GGA CCA GGA TTT ACT GGT GGG GAC TTA GTT AGA

505 510 515 520
Leu Asn Ser Ser Gly Asn Asn Ile Gln Asn Arg Gly Tyr Ile Glu Val Pro Ile His Phe
TTA AAT AGT AGT GGA AAT AAC ATT CAG AAT AGA GGG TAT ATT GAA GTT CCA ATT CAC TTC

525 530 535 540
Pro Ser Thr Ser Thr Arg Tyr Arg Val Arg Val Arg Tyr Ala Ser Val Thr Pro Ile His
CCA TCG ACA TCT ACC AGA TAT CGA GTT CGT GTA CGG TAT GCT TCT GTA ACC CCG ATT CAC

545 550 555 560
Leu Asn Val Asn Trp Gly Asn Ser Ser Ile Phe Ser Asn Thr Val Pro Ala Thr Ala Thr
CTC AAC GTT AAT TGG GGT AAT TCA TCC ATT TTT TCC AAT ACA GTA CCA GCT ACA GCT ACG

565 570 575 580
Ser Leu Asp Asn Leu Gln Ser Ser Asp Phe Gly Tyr Phe Glu Ser Ala Asn Ala Phe Thr
TCA TTA GAT AAT CTA CAA TCA AGT GAT TTT GGT TAT TTT GAA AGT GCC AAT GCT TTT ACA

585 590 595 600
Ser Ser Leu Gly Asn Ile Val Gly Val Arg Asn Phe Ser Gly Thr Ala Gly Val Ile Ile
TCT TCA TTA GGT AAT ATA GTA GGT GTT AGA AAT TTT AGT GGG ACT GCA GGA GTG ATA ATA

605 610 615 620
Asp Arg Phe Glu Phe Ile Pro Val Thr Ala Thr Leu Glu Ala Glu Tyr Asn Leu Glu Arg
GAC AGA TTT GAA TTT ATT CCA GTT ACT GCA ACA CTC GAG GCT GAA TAT AAT CTG GAA AGA

625 630 635 640
Ala Gln Lys Ala Val Asn Ala Leu Phe Thr Ser Thr Asn Gln Leu Gly Leu Lys Thr Asn
GCG CAG AAG GCG GTG AAT GCG CTG TTT ACG TCT ACA AAC CAA CTA GGG CTA AAA ACA AAT

645 650 655 660
Val Thr Asp Tyr His Ile Asp Gln Val Ser Asn Leu Val Thr Tyr Leu Ser Asp Glu Phe
GTA ACG GAT TAT CAT ATT GAT CAA GTG TCC AAT TTA GTT ACG TAT TTA TCG GAT GAA TTT

665 670 675 680
Cys Leu Asp Glu Lys Arg Glu Leu Ser Glu Lys Val Lys His Ala Lys Arg Leu Ser Asp
TGT CTG GAT GAA AAG CGA GAA TTG TCC GAG AAA GTC AAA CAT GCG AAG CGA CTC AGT GAT

685 690 695 700
Glu Arg Asn Leu Leu Gln Asp Ser Asn Phe Lys Asp Ile Asn Arg Gln Pro Glu Arg Gly
GAA CGC AAT TTA CTC CAA GAT TCA AAT TTC AAA GAC ATT AAT AGG CAA CCA GAA CGT GGG

705 710 715 720
Trp Gly Gly Ser Thr Gly Ile Thr Ile Gln Gly Gly Asp Asp Val Phe Lys Glu Asn Tyr
TGG GGC GGA AGT ACA GGG ATT ACC ATC CAA GGA GGG GAT GAC GTA TTT AAA GAA AAT TAC

725 730 735 740
Val Thr Leu Ser Gly Thr Phe Asp Glu Cys Tyr Pro Thr Tyr Leu Tyr Gln Lys Ile Asp
GTC ACA CTA TCA GGT ACC TTT GAT GAG TGC TAT CCA ACA TAT TTG TAT CAA AAA ATC GAT

745 750 755 760
Glu Ser Lys Leu Lys Ala Phe Thr Arg Tyr Gln Leu Arg Gly Tyr Ile Glu Asp Ser Gln
GAA TCA AAA TTA AAA GCC TTT ACC CGT TAT CAA TTA AGA GGG TAT ATC GAA GAT AGT CAA

765 770 775 780
Asp Leu Glu Ile Tyr Leu Ile Arg Tyr Asn Ala Lys His Glu Thr Val Asn Val Pro Gly
GAC TTA GAA ATC TAT TTA ATT CGC TAC AAT GCA AAA CAT GAA ACA GTA AAT GTG CCA GGT

785 790 795 800
Thr Gly Ser Leu Trp Pro Leu Ser Ala Gln Ser Pro Ile Gly Lys Cys Gly Glu Pro Asn
ACG GGT TCC TTA TGG CCG CTT TCA GCC CAA AGT CCA ATC GGA AAG TGT GGA GAG CCG AAT

805 810 815 820
Arg Cys Ala Pro His Leu Glu Trp Asn Pro Asp Leu Asp Cys Ser Cys Arg Asp Gly Glu
CGA TGC GCG CCA CAC CTT GAA TGG AAT CCT GAC TTA GAT TGT TCG TGT AGG GAT GGA GAA

825 830 835 840
Lys Cys Ala His His Ser His His Phe Ser Leu Asp Ile Asp Val Gly Cys Thr Asp Leu
AAG TGT GCC CAT CAT TCG CAT CAT TTC TCC TTA GAC ATT GAT GTA GGA TGT ACA GAC TTA

845 850 855 860
Asn Glu Asp Leu Gly Val Trp Val Ile Phe Lys Ile Lys Thr Gln Asp Gly His Ala Arg
AAT GAG GAC CTA GGT GTA TGG GTG ATC TTT AAG ATT AAG ACG CAA GAT GGG CAC GCA AGA

865 870 875 880
Leu Gly Asn Leu Glu Phe Leu Glu Glu Lys Pro Leu Val Gly Glu Ala Leu Ala Arg Val
CTA GGG AAT CTA GAG TTT CTC GAA GAG AAA CCA TTA GTA GGA GAA GCG CTA GCT CGT GTG

885 890 895 900
Lys Arg Ala Glu Lys Lys Trp Arg Asp Lys Arg Glu Lys Leu Glu Trp Glu Thr Asn Ile
AAA AGA GCG GAG AAA AAA TGG AGA GAC AAA CGT GAA AAA TTG GAA TGG GAA ACA AAT ATC

905 910 915 920
Val Tyr Lys Glu Ala Lys Glu Ser Val Asp Ala Leu Phe Val Asn Ser Gln Tyr Asp Gln
GTT TAT AAA GAG GCA AAA GAA TCT GTA GAT GCT TTA TTT GTA AAC TCT CAA TAT GAT CAA

925 930 935 940
Leu Gln Ala Asp Thr Asn Ile Ala Met Ile His Ala Ala Asp Lys Arg Val His Ser Ile
TTA CAA GCG GAT ACG AAT ATT GCC ATG ATT CAT GCG GCA GAT AAA CGT GTT CAT AGC ATT

945 950 955 960
Arg Glu Ala Tyr Leu Pro Glu Leu Ser Val Ile Pro Ala Val Asn Ala Ala Ile Phe Glu
CGA GAA GCT TAT CTG CCT GAG CTG TCT GTG ATT CCG GCT GTC AAT GCG GCT ATT TTT GAA

965 970 975 980
Glu Leu Glu Gly Arg Ile Phe Thr Ala Phe Ser Leu Tyr Asp Ala Arg Asn Val Ile Lys
GAA TTA GAA GGG CGT ATT TTC ACT GCA TTC TCC CTA TAT GAT GCG AGA AAT GTC ATT AAA

985 990 995 1000
Asn Gly Asp Phe Asn Asn Gly Leu Ser Cys Trp Asn Val Lys Gly His Val Asp Val Glu
AAT GGT GAT TTT AAT AAT GGC TTA TCC TGC TGG AAC GTG AAA GGG CAT GTA GAT GTA GAA

1005 1010 1015 1020
Glu Gln Asn Asn His Arg Ser Val Leu Val Val Pro Glu Trp Glu Ala Glu Val Ser Gln
GAA CAA AAC AAC CAC CGT TCG GTC CTT GTT GTT CCG GAA TGG GAA GCA GAA GTG TCA CAA

1025 1030 1035 1040
Glu Val Arg Val Cys Pro Gly Arg Gly Tyr Ile Leu Arg Val Thr Ala Tyr Lys Glu Gly
GAA GTT CGT GTC TGT CCG GGT CGT GGC TAT ATC CTT CGT GTC ACA GCG TAC AAG GAG GGA

1045 1050 1055 1060
Tyr Gly Glu Gly Cys Val Thr Ile His Glu Ile Glu Asn Asn Thr Asp Glu Leu Lys Phe
TAT GGA GAA GGT TGC GTA ACC ATT CAT GAG ATC GAG AAC AAT ACA GAC GAA CTG AAG TTT

1065 1070 1075 1080
Ser Asn Cys Val Glu Glu Glu Val Tyr Pro Asn Asn Thr Val Thr Cys Asn Asp Tyr Thr
AGC AAC TGT GTA GAA GAG GAA GTA TAT CCA AAC AAC ACG GTA ACG TGT AAT GAT TAT ACT

1085 1090 1095 1100
Ala Thr Gln Glu Glu Tyr Glu Ala Thr Tyr Thr Ser Arg Asn Arg Gly Tyr Asp Gly Ala
GCG ACT CAA GAA GAA TAT GAG GCT ACG TAC ACT TCT CGT AAT CGA GGA TAT GAC GGA GCC

1105 1110 1115 1120
Tyr Glu Ser Asn Ser Ser Val Pro Ala Asp Tyr Ala Ser Ala Tyr Glu Glu Lys Ala Tyr
TAT GAA AGC AAT TCT TCT GTA CCA GCT GAT TAT GCA TCA GCC TAT GAA GAA AAA GCA TAT

1125 1130 1135 1140
Thr Asp Gly Arg Arg Asp Asn Pro Cys Glu Ser Asn Arg Gly Tyr Gly Asp Tyr Thr Pro
ACA GAT GGA CGA AGA GAC AAT CCT TGT GAA TCT AAC AGA GGA TAT GGG GAT TAC ACA CCA

1145 1150 1155 1160
Leu Pro Ala Gly Tyr Val Thr Lys Glu Leu Glu Tyr Phe Pro Glu Thr Asp Lys Val Trp
CTA CCA GCT GGC TAT GTG ACA AAA GAA TTA GAG TAC TTC CCA GAA ACC GAT AAG GTA TGG

1165 1170 1175 1180
Ile Glu Ile Gly Glu Thr Glu Gly Thr Phe Ile Val Asp Ser Val Glu Leu Leu Leu Met
ATT GAG ATC GGA GAA ACG GAA GGA ACA TTC ATC GTG GAC AGC GTG GAA TTA CTT CTT ATG

1185 1190 1195 1200
Glu Glu ***
GAG GAA TAA TAT ATG CTT TAA AAT GTA AGG TGT GCA AAT AAA GAA TGA TTA CTG ACT TGT

1205 1210 1215 1220
ATT GAC AGA TAA ATA AGG AAA TTT TTA TAT GAA TAA AAA ACG GGC ATC ACT CTT AAA AGA

1225 1230 1235
ATG ATG TCC GTT TTT TGT ATG ATT TAA CGA GTG ATA TTT AAA TGT TTT TTT GCG AAG G

## Claims (Claims for the following Contracting State(s): AT, BE, CH, DE, FR, GB, GR, IT, LU, NL, SE)

1. DNA encoding a Bacillus thuringiensis toxin having the amino-acid sequence shown in the Chart.

2. DNA according to claim 1, having the nucleotide sequence shown in the Chart.

3. DNA according to claim 1 or claim 2, wherein the nucleotide sequence terminates at the stop codon.

4. A toxin active against lepidopteran insects, having the amino-acid sequence shown in the Chart.

5. A recombinant DNA transfer vector comprising DNA according to any of claims 1 to 3.

6. A prokaryotic or eukaryotic host to which a DNA transfer vector according to claim 5 has been transferred and replicated.

7. A microorganism capable of expressing a Bacillus thuringiensis toxin having the amino-acid sequence shown in the Chart.

8. A microorganism according to claim 7, which is a species of Pseudomonas, Azobacter, Erwinia, Serratia, Klebsiella, Rhizobium, Rhodopseudomonas, Methylophilius, Agrobacterium, Acetobacter or Alcaligenes; a prokaryote selected from Enterobacteriaceae, Bacillaceae, Rhizobiaceae, Spirillaceae, Lactobacillaceae, Pseudomonadaceae, Azotobacteraceae and Nitrobacteraceae; or a lower eukaryote selected from Phycomycetes, Ascomycetes and Basidiomycetes.

9. A microorganism according to claim 7, which is Pseudomonas fluorescens.

10. A microorganism according to claim 9, which is Pseudomonas fluorescens (pM2,16-11), having the identifying characteristic of NRRL B-18292.

11. A microorganism according to claim 7, which is a pigmented bacterium, yeast or fungus.

12. A microorganism according to any of claims 7 to 11, which is pigmented and phylloplane-adherent.

13. Substantially intact cells of a unicellular microorganism according to any of claims 7 to 12, containing the toxin.

14. Cells according to claim 13, as obtained by treatment with iodine or other chemical or physical means to prolong the insecticidal activity in the environment.

15. A method for controlling lepidopteran insects, which comprises administering to the insects or to their environment a microorganism or cells according to any of claims 7 to 14.

16. A method according to claim 15, wherein administration is to the rhizosphere, to the phylloplane, or to a body of water.

17. The plasmid pM2,16-11 as available in Pseudomonas fluorescens (pM2,16-11), having the identifying characteristic of NRRL B-18292.

## Claims (Claims for the following Contracting State(s): ES)

1. A method for controlling lepidopteran insects, which comprises administering to the insects or to their environment a microorganism capable of expressing a Bacillus thuringiensis toxin having the amino-acid sequence shown in the Chart.

2. A method according to claim 1, wherein the microorganism is a species of Pseudomonas, Azobacter, Erwinia, Serratia, Klebsiella, Rhizobium, Rhodopseudomonas, Methylophilius, Agrobacterium, Acetobacter or Alcaligenes; a prokaryote selected from Enterobacteriaceae, Bacillaceae, Rhizobiaceae, Spirillaceae, Lactobacillaceae, Pseudomonadaceae, Azotobacteraceae and Nitrobacteraceae; or a lower eukaryote selected from Phycomycetes, Ascomycetes and Basidiomycetes.

3. A method according to claim 1, wherein the microorganism is Pseudomonas fluorescens.

4. A method according to claim 3, wherein the microorganism is Pseudomonas fluorescens (pM2,16-11), having the identifying characteristic of NRRL B-18292.

5. A method according to claim 1, wherein the microorganism is a pigmented bacterium, yeast or fungus.

6. A method according to any of claims 1 to 5, wherein the microorganism is pigmented and phylloplane-adherent.

7. A method for controlling lepidopteran insects, which comprises administering to the insects or to their environment substantially intact cells of a unicellular microorganism as defined in any preceding claim, containing the toxin.

8. A method according to claim 7, wherein the cells are obtained by treatment with iodine or other chemical or physical means to prolong the insecticidal activity in the environment.

9. A method according to any preceding claim, wherein administration is to the rhizosphere, to the phylloplane, or to a body of water.

## Patentansprüche (Patentansprüche für folgende(n) Vertragsstaat(en): AT, BE, CH, DE, FR, GB, GR, IT, LU, NL, SE)

1. DNA, die für ein Bacillus thuringiensis-Toxin mit der in der TABELLE gezeigten Aminosäuresequenz kodiert.

2. DNA nach Anspruch 1, mit der in der TABELLE gezeigten Nukleotidsequenz.

3. DNA nach Anspruch 1 oder 2, bei der die Nukleotidsequenz am Stopcodon endet.

4. Gegen Lepidoptera-Insekten aktives Toxin mit der in der TABELLE gezeigten Aminosäuresequenz.

5. Rekombinanter DNA-Transfervektor, der DNA nach irgendeinem der Ansprüche 1 bis 3 umfaßt.

6. Prokaryotischer oder eukaryotischer Wirt, in den ein DNA-Transfervektor nach Anspruch 5 transferiert und repliziert worden ist.

7. Mikroorganismus, der imstande ist, ein Bacillus thuringiensis-Toxin mit der in der TABELLE gezeigten Aminosäuresequenz zu exprimieren.

8. Mikroorganismus nach Anspruch 7, der eine Spezies ist von Pseudomonas, Azobacter, Erwinia, Serratia, Klebsiella, Rhizobium, Rhodopseudomonas, Methylophilius, Agrobacterium, Acetobacter oder Alcaligenes; ein Prokaryot ausgewählt aus Enterobacteriaceae, Bacillaceae, Rhizobiaceae, Spirillaceae, Lactobacillaceae, Pseudomonadaceae, Azotobacteraceae und Nitrobacteraceae; oder ein niederer Eukaryot, ausgewählt aus Phycomycetes, Ascomycetes und Basidiomycetes.

9. Mikroorganismus nach Anspruch 7, der Pseudomonas fluorescens ist.

10. Mikroorganismus nach Anspruch 9, der Pseudomonas fluorescens (pM2,16-11) mit den kennzeichnenden Merkmalen von NRRL B-18292 ist.

11. Mikroorganismus nach Anspruch 7, der ein pigmentiertes Bakterium, Hefe oder ein Pilz ist.

12. Mikroorganismus nach irgendeinem der Ansprüche 7 bis 11, der pigmentiert ist und Phyllooberflächenadhärent.

13. Im wesentlichen intakte Zellen eines einzelligen Mikroorganismus nach irgendeinem der Ansprüche 7 bis 12, der das Toxin enthält.

14. Zellen nach Anspruch 13, wie erhalten durch Behandlung mit Iod oder anderen chemischen oder physikalischen Mitteln, um die insektizide Aktivität in der Umwelt zu verlängern.

15. Verfahren zur Kontrolle eines Lepidoptera-Insektenschädlings, welches das Ausbringen eines Mikroorganismus oder Zellen nach irgendeinem der Ansprüche 7 bis 14 an die Insekten oder in deren Umwelt umfaßt.

16. Verfahren nach Anspruch 15, worin die Ausbringung in der Rhizosphäre, der Phyllooberfläche oder einem Wasserkörper erfolgt.

17. Plasmid pM2,16-11, wie erhältlich in Pseudomonas fluorescens (pM2,16-11) mit den kennzeichnenden Merkmalen von NRRL B-18292).

## Patentansprüche (Patentansprüche für folgende(n) Vertragsstaat(en): ES)

1. Verfahren zur Kontrolle von Lepidoptera-Insekten, welches das Ausbringen eines Mikroorganismus, der imstande ist, ein Bacillus thuringiensis-Toxin mit der in der TABELLE gezeigten Aminosäuresequenz zu exprimieren, an die Insekten oder in deren Umwelt umfaßt.

2. Verfahren nach Anspruch 1, worin der Mikroorganismus eine Spezies ist von Pseudomonas, Azobacter, Erwinia, Serratia, Klebsiella, Rhizobium, Rhodopseudomonas, Methylophilius, Agrobacterium, Acetobacter oder Alcaligenes; ein Prokaryot ausgewählt aus Enterobacteriaceae, Bacillaceae, Rhizobiaceae, Spirillaceae, Lactobacillaceae, Pseudomonadaceae, Azotobacteraceae und Nitrobacteraceae; oder ein niederer Eukaryot, ausgewählt aus Phycomycetes, Ascomycetes und Basidiomycetes.

3. Verfahren nach Anspruch 1, worin der Mikroorganismus Pseudomonas fluorescens ist.

4. Verfahren nach Anspruch 3, worin der Mikroorganismus Pseudomonas fluorescens (pM2,16-11) mit den kennzeichnenden Merkmalen von NRRL B-18292 ist.

5. Verfahren nach Anspruch 1, worin der Mikroorganismus ein pigmentiertes Bakterium, Hefe oder ein Pilz ist.

6. Verfahren nach irgendeinem der Ansprüche 1 bis 5, worin der Mikroorganismus pigmentiert ist und Phyllooberflächen-adhärent.

7. Verfahren zur Kontrolle von Lepidoptera-Insekten, welches das Ausbringen von im wesentlichen intakten Zellen eines einzelligen Mikroorganismus nach irgendeinem der vorhergehenden Ansprüche, der das Toxin enthält, an die Insekten oder in deren Umwelt umfaßt.

8. Verfahren nach Anspruch 7, worin die Zellen erhalten werden durch Behandlung mit Iod oder anderen chemischen oder physikalischen Mitteln, um die insektizide Aktivität in der Umwelt zu verlängern.

9. Verfahren nach irgendeinem vorhergehenden Anspruch, worin die Ausbringung in der Rhizosphäre, der Phyllooberfläche oder einem Wasserkörper erfolgt.

## Revendications (Revendications pour l'(les) Etat(s) contractant(s) suivant(s): AT, BE, CH, DE, FR, GB, GR, IT, LU, NL, SE)

1. ADN codant pour une toxine de *Bacillus thuringiensis* ayant la séquence d'aminoacides présentée dans la planche.

2. ADN selon la revendication 1, ayant la séquence de nucléotides présentée dans la planche.

3. ADN selon la revendication 1 ou 2, dans lequel la séquence de nucléotides se termine au codon d'arrêt.

4. Toxine active contre les insectes lépidoptères, ayant la séquence d'aminoacides présentée dans la planche.

5. Vecteur de transfert d'ADN recombiné comprenant un ADN selon l'une quelconque des revendications 1 à 3.

6. Hôte procaryote ou eucaryote dans lequel a été transféré et répliqué un vecteur de transfert d'ADN selon la revendication 5.

7. Microorganisme capable d'exprimer une toxine de *Bacillus thuringiensis* ayant la séquence d'aminoacides présentée dans la planche.

8. Microorganisme selon la revendication 7, qui est une espèce de *Pseudomonas, Azobacter*, *Erwinia, Serratia*, *Klebsiella, Rhizobium, Rhodopseudomonas*, *Methylophilius, Agrobacterium*, *Acetobacter* ou *Alcaligenes*; un procaryote choisi parmi les Enterobacteriaceae, les Bacillaceae, les Rhizobiaceae, les Spirillaceae, les Lactobacillaceae, les Pseudomonadaceae, les Azotobacteraceae et les Nitrobacteraceae; ou un eucaryote inférieur choisi parmi les Phycomycètes, les Ascomycètes et les Basidiomycètes.

9. Microorganisme selon la revendication 7, qui est *Pseudomonas fluorescens*.

10. Microorganisme selon la revendication 9, qui est *Pseudomonas fluorescens* (pM2,16-11), ayant les caractères d'identification de NRRL B-18292.

11. Microorganisme selon la revendication 7, qui est une bactérie, une levure ou une champignon pigmenté(e).

12. Microorganisme selon l'une quelconque des revendications 7 à 11, qui est pigmenté et qui adhère à la surface des feuilles.

13. Cellules essentiellement entières d'un microorganisme unicellulaire selon l'une quelconque des revendications 7 à 12, contenant la toxine.

14. Cellules selon la revendication 13, telle qu'elle est obtenue par traitement avec de l'iode ou un autre moyen chimique ou physique destiné à prolonger l'activité insecticide dans l'environnement.

15. Méthode de lutte contre les insectes lépidoptères, qui comprend l'administration aux insectes ou à leur environnement d'un microorganisme ou de cellules selon l'une quelconque des revendications 7 à 14.

16. Méthode selon la revendication 15, dans laquelle on effectue l'administration à la rhizosphère, à la surface des feuilles ou à un organisme aquatique.

17. Plasmide pM2,16-11, tel qu'il se trouve dans *Pseudomonas fluorescens* (pM2,16-11), ayant les caractères d'identification de NRRL B-18292.

## Revendications (Revendications pour l'(les) Etat(s) contractant(s) suivant(s): ES)

1. Méthode de lutte contre les insectes lépidoptères, qui comprend l'administration aux insectes ou à leur environnement d'un microorganisme capable d'exprimer une toxine de *Bacillus thuringiensis* ayant la séquence d'aminoacides présentée dans la planche.

2. Méthode selon la revendication 1, dans laquelle le microorganisme est une espèce de *Pseudomonas, Azobacter*, *Erwinia*, *Serratia*, *Klebsiella*, *Rhizobium*, *Rhodopseudomonas*, *Methylophilius*, *Agrobacterium*, *Acetobacter* ou *Alcaligenes*; un procaryote choisi parmi les Enterobacteriaceae, les Bacillaceae, les Rhizobiaceae, les Spirillaceae, les Lactobacillaceae, les Pseudomonadaceae, les Azotobacteraceae et les Nitrobacteraceae; ou un eucaryote inférieur choisi parmi les Phycomycètes, les Ascomycètes et les Basidiomycètes.

3. Méthode selon la revendication 1, dans laquelle le microorganisme est *Pseudomonas fluorescens*.

4. Méthode selon la revendication 3, dans laquelle le microorganisme est *Pseudomonas fluorescens* (pM2,16-11), ayant les caractères d'identification de NRRL B-18292.

5. Méthode selon la revendication 1, dans laquelle le microorganisme est une bactérie, une levure ou une champignon pigmenté(e).

6. Méthode selon l'une quelconque des revendications 1 à 5, dans laquelle le microorganisme est pigmenté et adhère à la surface des feuilles.

7. Méthode de lutte contre les insectes lépidoptères, qui comprend l'administration aux insectes ou à leur environnement de cellules essentiellement entières d'un microorganisme unicellulaire tel que défini dans l'une quelconque des revendications précédentes, contenant la toxine.

8. Méthode selon la revendication 7, dans laquelle les cellules sont obtenues par traitement avec de l'iode ou un autre moyen chimique ou physique destiné à prolonger l'activité insecticide dans l'environnement.

9. Méthode selon l'une quelconque des revendications précédentes, dans laquelle on effectue l'administration à la rhizosphère, à la surface des feuilles ou à un organisme aquatique.
